# EUROPEAN PATENT SPECIFICATION

(11) **EP 1 784 235 B1**
(45) Date of publication and mention of the grant of the patent: **17.11.2010**
(21) Application number: 05794231.0
(22) Date of filing: 15.08.2005
(51) Int. Cl.: A61M 5/145, A61M 5/142

(54) **LIQUID DISPENSING DEVICE**
FLÜSSIGKEITSABGABEVORRICHTUNG
DISPOSITIF DE DISTRIBUTION DE LIQUIDE

(30) Priority: 16.08.2004 GB 0418256
(43) Date of publication of application: 16.05.2007
(73) Proprietor: The Technology Partnership Plc, Melbourn, Royston, Hertfordshire SG8 6EE (GB)
(72) Inventor: CARR, Matthew, John, Cambridge CB4 3EW (GB); McCRONE, James, Edward, Cambridge CB2 2HU (GB)
(74) Representative: Smee, Anthony James Michael
(86) International application number: PCT/GB2005/003184
(87) International publication number: WO 2006/018617

(56) References cited:
- WO-A-03/000318
- US-A1- 2004 078 028
- US-A1- 2004 153 032
- US-B1- 6 607 495

## Description

This invention relates to a liquid dispensing device and, in particular, to a device that can be used in drug delivery infusion, or in consumer dispensing products for the metered dispensing of materials such as pastes or creams. It can also be used in microfluidic applications.

Recent advances in the areas of microfluidics and dispensing demand precise control of extremely small volumes of fluid and it is in this area that the present invention has particular relevance. In microfluidics, drug infusion and dispensing, flow systems are required which have a small physical size, but the physical size of existing systems is currently limited by the complexity and spatial requirement of available liquid delivery devices.

Commonly, for example, infusion devices delivering drugs, such as insulin, operate using a motor driven syringe. This principle has been known since at least 1976. These systems employ an encoded motor, a reduction gear box and a lead screw to drive a plunger along the bore of a reservoir. The plunger in turn forces liquid out of the reservoir. The systems are relatively complex, expensive and large. The size and weight of these systems has thus far prohibited the development of a skin worn delivery device. Instead, the infusion site is linked via a length of tubing to a separate unit carried in the pocket or on the belt of the user. This length of tubing introduces volumetric compliance issues, occlusion detection delays and substantially increases the time taken to prime the system. In addition, separating the infusion site from the delivery device in this way makes the system far more cumbersome in use.

Accordingly, the present invention aims to provide a liquid dispensing device which overcomes the above problems and which can be produced in a sufficiently small size that it can be worn on the skin.

US 2004/078028 discloses the features of the preamble of claim 1 and describes a liquid dispensing device comprising:
a chamber in which a liquid to be dispensed is retained in use, the chamber having a first and a second opening;
a one way valve retained in the first opening to allow liquid flow only out of the chamber; and
a closure slidably retained in the chamber to close the second opening, the closure including
an actuator having a flexible element.

According to the present invention, such a device is characterised in that the flexible element upon actuation, is caused to extend into the chamber reducing its volume and thereby causing liquid to be expelled from the chamber through the valve,
wherein, when actuation is stopped, the closure is caused to slide in the chamber so as to reduce the volume of the chamber by the amount of the dispensed liquid.

Accordingly, the present invention as claimed provides a liquid dispensing device which can be manufactured in a sufficiently small size such that it can be worn on the skin close to the injection site, thereby removing the need for lengthy tubes. In addition, by the use of a simple actuator, rather than motors, gear boxes and lead screws, the present invention ensures that the complexity and weight of the device is considerably reduced.

In this specification, the term "liquid" covers creams, pastes, suspensions and any other flowable substance.

The flexible element may be a diaphragm or other suitable thin construction, typically formed from an elastomeric material such as rubber. The actuator may be mounted on the flexible element.

The actuator may be connected to a microprocessor or other programmable control device, so that the operation of the actuator can be precisely controlled. Such a control device would also permit the device to be operated automatically, i.e. without regular supervision by a doctor, nurse or the like.

The chamber may have a reduced diameter portion adjacent the valve. In one embodiment, the actuator includes a piezoelectric element. The piezoelectric element is preferably arranged such that, upon actuation, the flexible element extends only into the chamber and that, when actuation is stopped, it returns to its normal non-extended state. The flexible element may be the piezoelectric element itself or may include the piezoelectric element as a part of its construction.

Alternatively, in a second embodiment, the actuator includes a shape-memory alloy element which is preferably connected to the flexible element. The shape-memory alloy element is preferably connected to the flexible element by a plurality of hooks. The shape-memory alloy element is preferably arranged such that, upon actuation, the flexible element extends only into the chamber and that, when actuation is stopped, it returns to its normal non-extended state.

Example of the present invention will now be described with reference to the accompanying drawings, in which:
Fig. 1 shows a schematic side view of one example of a device according to the present invention;
Figs. 2A to 2C show various steps in the operation of the device of Fig. 1;
Fig. 3 shows one example of a closure for use in the device of Fig. 1;
Fig. 4 shows a second example of a closure for use in the device of Fig. 1; and
Fig. 5 shows two examples of elements for preventing movement of the closure in an unwanted direction.

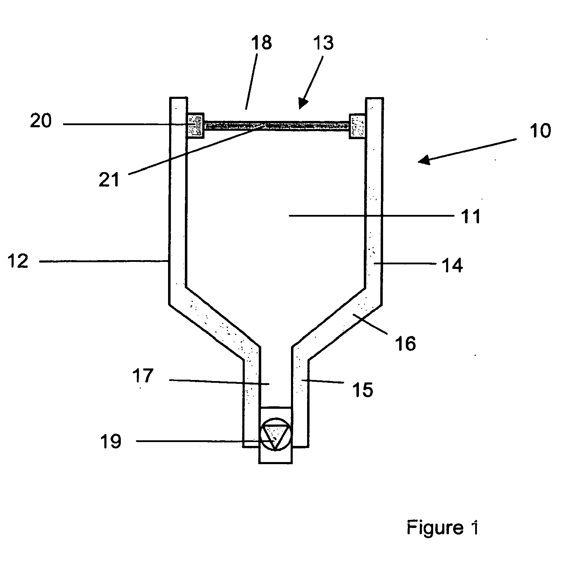

Fig. 1 shows a liquid dispensing device 10 having a chamber 11 defined by outer walls 12 and a closure 13. The outer walls 12 are shaped so as to define a relatively large cylindrical portion 14, a relatively narrow cylindrical portion 15 and a tapered portion 16 connecting the relatively large and relatively small cylindrical portions. The chamber 11 has a first opening 17 and a second opening 18. A one-way valve 19 is retained within the first opening to permit fluid flow only out of the chamber 11. The closure 13 is retained in the second opening 18 and is provided with a sliding seal 20 between it and the inner surface of the wall 12.

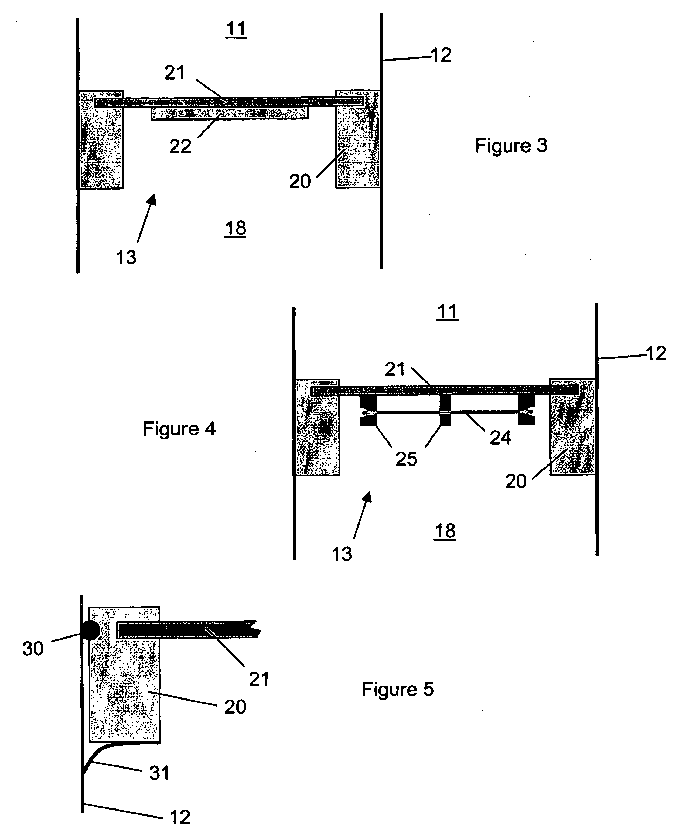

In use, a volume of liquid (not shown) is retained within the chamber 11 and the closure 13 is placed in contact with the volume of liquid so that there is no free space in the chamber. The closure 13 is provided with a flexible element 21 and an actuator (see Figs. 3 and 4), such as a piezoelectric element or a shape-memory alloy element.

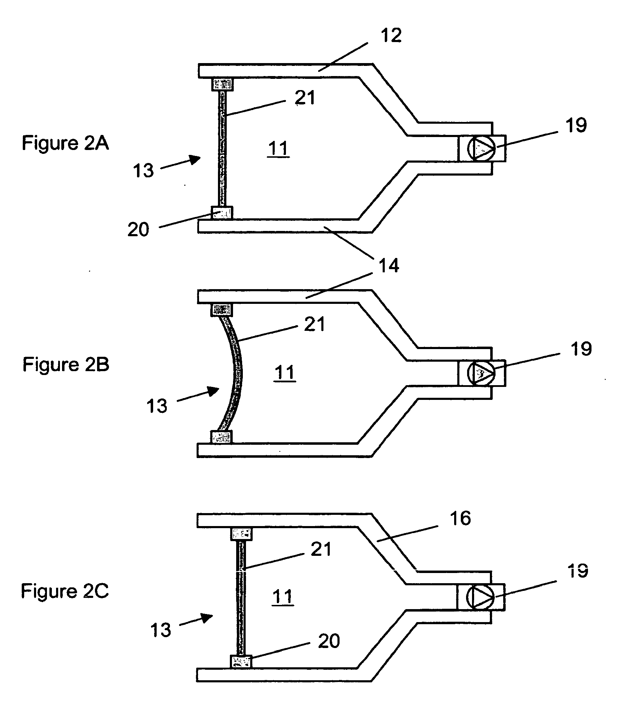

Referring now to Figs. 2A to 2C, Fig. 2A shows the device of Fig. 1 in an inactive state. Upon actuation of the actuator, the flexible element 13 is caused to extend into the chamber 11, as seen in Fig. 2B. This reduces the volume of the chamber 11 and, as a result, a small volume of liquid is thus expelled through the valve 19. When actuation is stopped, the pressure within the chamber 11 is reduced, as the flexible element tries to return to its non-extended state and the check valve prevents air from entering the device. The difference between the ambient pressure outside the chamber and the lower pressure within the chamber 11 causes the closure 13 to move along the inner surface of the wall 12. This reduces the volume of the chamber 11 and equalizes the pressure within the chamber with the ambient outside pressure. In doing so, as can be seen in Fig. 2C, the seal 20 slides along the inner surface of the wall 12 such that the closure 13 advances an incremental distance towards the valve 19 and the device is primed for dispensing a further volume of fluid upon further actuation of the actuator.

Fig. 3 shows one example of a closure for use in the device of Fig. 1. In this example, the closure 13 comprises a flexible element 21 which is mounted in a sliding seal 20, thereby closing opening 18. An actuator 22, comprising a piezoelectric element, is attached to the flexible element 21. When an electric field is applied to the piezoelectric element 22, it is caused to contract, thereby reacting against the flexible element 21 and causing the flexible element 21 to extend into the chamber 11. When the electric field is removed from the piezoelectric element, it returns to its original shape, thereby causing the flexible element to return to its original shape. The flexible element 21 may also be resilient and the resilience of the element 21 can assist in returning the piezoelectric element to its original shape.

A further example of a closure is shown in Fig. 4, in which a flexible element 21 is mounted in a sliding seal 20, thereby closing opening 18. In this example, the actuator consists of a length of shape-memory alloy wire 24, formed into a loop, and secured around a number of hooks 25 which are connected to the flexible element 21. When a suitable electric current is passed through the wire 24, it contracts in length significantly, thereby causing the hooks 25 to be drawn towards each other and the flexible element to bow into the chamber 11. When the electric current is stopped, the wire returns to its original length and the flexible element returns to its original shape. Again, the flexible element may be resilient, to assist in the return to the original state.

In both the arrangements shown in Figs. 3 and 4, due to provision of the one-way valve and the lower than ambient pressure within the chamber 11 after liquid has been expelled, the actuator and flexible element return to their original shape by causing the sliding seal 20 to move along the inner surface of wall 12 towards the fist opening.

As shown in Fig. 5, the liquid dispensing device of the present invention may be provided with one or more elements that engage with the wall 12 of the chamber to prevent, or at least hinder, movement of closure 13 away from the one way valve 19. In one example, an O-ring 30 may be used, as well as seal 20, and the O-ring may be shaped or formed so as to prevent movement of the closure 13 away from the first opening, i.e. downwards in Fig. 5. Additionally or alternatively, one or more barbs 31, typically formed from a resilient material, may be provided and which extend from the seal 20 to engage with the inner surface of the wall 12. Movement of the closure towards the first opening, i.e. upwards in Fig. 5, is permitted as the barbs slide along the inner surface of the wall 12, but when movement is attempted in the opposite direction, i.e. downwards in Fig. 5, the barbs 31 dig into the inner surface of the wall 12.

## Claims

1. A liquid dispensing device (10) comprising:
a chamber (11) in which a liquid to be dispensed is retained in use, the chamber having a first (17) and a second opening (18);
a one way valve (19) retained in the first opening to allow liquid flow only out of the chamber;
a closure (13) slidably retained in the chamber to close the second opening, the closure (13) including
an actuator (22,24) having a flexible element (21); **characterised in that**
the flexible element, upon actuation, is caused to extend into the chamber (11) reducing its volume and thereby causing liquid to be expelled from the chamber through the valve (19),
wherein, when actuation is stopped, the closure is caused to slide in the chamber so as to reduce the volume of the chamber by the amount of the dispensed liquid.

2. A liquid dispensing device (10) according to claim 1, wherein the actuator (22,24) includes a piezoelectric element (22).

3. A liquid dispensing device (10) according to claim 2, wherein the piezoelectric element (22) is arranged such that, upon actuation, it extends only into the chamber (13) and that, when actuation is stopped, it returns to its normal non-extended state.

4. A liquid dispensing device (10) according to either claim 2 or claim 3, wherein the piezoelectric element (22) is mounted on the flexible element (21).

5. A liquid dispensing device (10) according to either claim 2 or claim 3, wherein the piezoelectric element (22) is part or all of the flexible element (21).

6. A liquid dispensing device (10) according to claim 1, wherein the actuator (22) includes an element (24) formed from a shape-memory alloy.

7. A liquid dispensing device (10) according to claim 5, wherein the shape-memory alloy (24) is formed as a wire connected to the flexible member.

8. A liquid dispensing device (10) according to claim 7, wherein the wire is connected to the flexible member (21) by a plurality of hooks (25).

9. A liquid dispensing device (10) according to any one of the preceding claims, wherein the actuator (22,24) is connected to a microprocessor or other programmable control device.

10. A liquid dispensing device (10) according to any one of the preceding claims, wherein the chamber (11) has a reduced diameter portion adjacent the valve.

11. A liquid dispensing device (10) according to any one of the preceding claims, wherein the closure (13) includes a seal (20) between the flexible element (21) and the chamber (11).

12. A liquid dispensing device (10) according to any one of the preceding claims, further comprising at least one element (20,30,31) for engaging with the wall of the chamber to hinder movement of the closure away from the first opening.

13. A liquid dispensing device (10) according to claim 12, wherein the engaging element(s) is (are) a barb (31) mounted on the closure (13).

14. A liquid dispensing device (10) according to claim 12, wherein the engaging element is an O-ring seal (30) between the closure (13) and the wall of the chamber (11).

## Patentansprüche

1. Flüssigkeitsabgabevorrichtung (10), die Folgendes umfasst:
eine Kammer (11), in der eine abzugebende Flüssigkeit aktiv enthalten ist, wobei die Kammer eine erste (17) und eine zweite Öffnung hat (18);
ein Rückschlagventil (19), das in der ersten Öffnung enthalten ist, und nur einen Flüssigkeitsfluss aus der Kammer ermöglicht;
einen Verschluss (13), der schiebbar in der Kammer enthalten ist, um die zweite Öffnung zu schließen, wobei der Verschluss (13) Folgendes beinhaltet:
ein Betätigungsglied (22, 24), das ein flexibles Element (21) hat,
**dadurch gekennzeichnet, dass**
das flexible Element nach der Betätigung, sich in die Kammer (11) erweitert, sein Volumen reduziert und somit bewirkt, dass Flüssigkeit von der Kammer durch das Ventil (19) ausgestoßen wird,
wobei beim Stoppen der Betätigung der Verschluss bewirkt, dass er in der Kammer rutscht, so dass er das Volumen der Kammer um die Menge der abgegebenen Flüssigkeit reduziert.

2. Flüssigkeitsabgabevorrichtung (10) nach Anspruch 1, wobei das Betätigungsglied (22, 24) ein piezoelektrisches Element (22) beinhaltet.

3. Flüssigkeitsabgabevorrichtung (10) nach Anspruch 2, wobei das piezoelektrische Element (22) so arrangiert ist, dass es sich bei Betätigung nur in die Kammer (13) erstreckt und dass es beim Stoppen der Betätigung zu seinem normalen nicht erweiterten Zustand zurückkehrt.

4. Flüssigkeitsabgabevorrichtung (10) nach entweder Anspruch 2 oder Anspruch 3, wobei das piezoelektrische Element (22) auf dem flexiblen Element (21) montiert ist.

5. Flüssigkeitsabgabevorrichtung (10) nach entweder Anspruch 2 oder Anspruch 3, wobei das piezoelektrische Element (22) ein Teil oder das gesamte flexible Element (21) ist.

6. Flüssigkeitsabgabevorrichtung (10) nach Anspruch 1, wobei das Betätigungsglied (22) ein Element (24) beinhaltet, das aus einer Formgedächtnislegierung geformt ist.

7. Flüssigkeitsabgabevorrichtung (10) nach Anspruch 5, wobei die Formgedächtnislegierung (24) als Draht geformt ist, der an das flexible Element angeschlossen ist.

8. Flüssigkeitsabgabevorrichtung (10) nach Anspruch 7, wobei der Draht an das flexible Element (21) mit einer Vielfalt von Haken (25) angeschlossen ist.

9. Flüssigkeitsabgabevorrichtung (10) nach einem der vorangehenden Ansprüche, wobei das Betätigungsglied (22, 24) an einem Mikroprozessor oder einem anderen Steuergerät angeschlossen ist.

10. Flüssigkeitsabgabevorrichtung (10) nach einem der vorangehenden Ansprüche, wobei die Kammer (11) einen Abschnitt mit reduziertem Durchmesser neben dem Ventil hat.

11. Flüssigkeitsabgabevorrichtung (10) nach einem der vorangehenden Ansprüche, wobei der Verschluss (13) eine Dichtung (20) zwischen dem flexiblen Element (21) und der Kammer (11) beinhaltet.

12. Flüssigkeitsabgabevorrichtung (10) nach einem der vorangehenden Ansprüche, die außerdem mindestens ein Element (20, 30, 31) umfasst, das in die Wand der Kammer eingreift, um eine Bewegung des Verschlusses von der ersten Öffnung weg verhindert.

13. Flüssigkeitsabgabevorrichtung (10) nach Anspruch 12, wobei das eingreifende Element (die Elemente) ein Widerhaken (31) ist, der auf dem Verschluss (13) montiert ist.

14. Flüssigkeitsabgabevorrichtung (10) nach Anspruch 12, wobei das eingreifende Element eine O-Ring-Dichtung (30) zwischen dem Verschluss (13) und der Wand der Kammer (11) ist.

## Revendications

1. Dispositif de distribution de liquide (10) comprenant :
une chambre (11) dans laquelle un liquide à distribuer est retenu en cours d'utilisation, ladite chambre ayant une première (17) et une seconde ouvertures (18) ;
une soupape unidirectionnelle (19) retenue dans la première ouverture pour permettre au liquide de s'écouler uniquement hors de la chambre ;
une fermeture (13) retenue par glissement dans la chambre pour fermer la seconde ouverture, ladite fermeture (13) incluant
un actionneur (22, 24) muni d'un élément flexible (21) ; **caractérisé en ce que**
l'élément flexible, lorsqu'il est actionné, est forcé de se prolonger dans la chambre (11) réduisant ainsi son volume et, par conséquent, provoquant l'expulsion du liquide contenu dans la chambre en le faisant passer par la soupape (19),
dans lequel, à la fin de l'actionnement, le glissement de la fermeture dans la chambre est causé afin de réduire le volume de la chambre par la quantité du liquide distribué.

2. Dispositif de distribution de liquide (10) selon la revendication 1, dans lequel l'actuateur (22, 24) inclut un élément piézoélectrique (22).

3. Dispositif de distribution de liquide (10) selon la revendication 2, dans lequel l'élément piézoélectrique (22) est disposé de manière telle que, lorsqu'il est actionné, il se prolonge uniquement dans la chambre (13) et que, à la fin de l'actionnement, il revienne à son état non prolongé normal.

4. Dispositif de distribution de liquide (10) selon la revendication 2 ou 3, dans lequel l'élément piézoélectrique (22) est monté sur l'élément flexible (21).

5. Dispositif de distribution de liquide (10) selon la revendication 2 ou 3, dans lequel l'élément piézoélectrique (22) fait partie, partiellement ou totalement, de l'élément flexible (21).

6. Dispositif de distribution de liquide (10) selon la revendication 1, dans lequel l'actionneur (22) inclut un élément (24) formé à partir d'un alliage à mémoire de forme.

7. Dispositif de distribution de liquide (10) selon la revendication 5, dans lequel l'alliage à mémoire de forme (24) est formé comme un fil métallique connecté au membre flexible.

8. Dispositif de distribution de liquide (10) selon la revendication 7, dans lequel le fil métallique est connecté au membre flexible (21) par une pluralité de crochets (25).

9. Dispositif de distribution de liquide (10) selon l'une quelconque des revendications précédentes, dans lequel l'actuateur (22, 24) est connecté à un microprocesseur ou à tout autre dispositif de contrôle programmable.

10. Dispositif de distribution de liquide (10) selon l'une quelconque des revendications précédentes, dans lequel la chambre (11) a une portion de diamètre réduite adjacente à la soupape.

11. Dispositif de distribution de liquide (10) selon l'une quelconque des revendications précédentes, dans lequel la fermeture (13) inclut un joint d'étanchéité (20) entre l'élément flexible (21) et la chambre (11).

12. Dispositif de distribution de liquide (10) selon l'une quelconque des revendications précédentes comprenant, en outre, au moins un élément (20, 30, 31) s'enclenchant dans la paroi de la chambre afin d'éviter que la fermeture ne s'éloigne de la première ouverture.

13. Dispositif de distribution de liquide (10) selon la revendication 12, dans lequel le(s) élément(s) s'engageant est(sont) un picot (31) monté sur la fermeture (13).

14. Dispositif de distribution de liquide (10) selon la revendication 12, dans lequel l'élément enclenchant est un joint torique (30) situé entre la fermeture (13) et la paroi de la chambre (11).
